# EUROPEAN PATENT APPLICATION

(11) **EP 3 666 750 A1**
(43) Date of publication of application: **17.06.2020**
(21) Application number: 18211435.5
(22) Date of filing: 10.12.2018
(51) Int. Cl.: C07C 51/09, C07C 59/245, A61K 31/20, A61P 3/06

(54) **CRYSTALLINE FORM OF BEMPEDOIC ACID**

(71) Applicant: Sandoz AG, 4056 Basel (CH)
(72) Inventor: NERDINGER, Sven, 6250 Kundl (AT); STEFINOVIC, Marijan, 6250 Kundl (AT); RAUCH, Erik, 66123 Saarbrücken (DE)
(74) Representative: Ter Meer Steinmeister & Partner

(57) **Abstract**

The present invention concerns crystalline bempedoic acid, a process for obtaining crystalline bempedoic acid, and pharmaceutical compositions comprising crystalline bempedoic acid.

## Description

### Field of the invention

The present invention relates to crystalline bempedoic acid. The present invention also relates to a novel process for the synthesis of crystalline bempedoic acid. Finally, the present invention is directed to a pharmaceutical preparation comprising crystalline bempedoic acid.

### Background of the invention

Bempedoic acid is a compound of Formula I 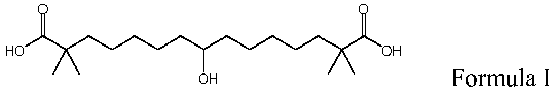 and has the IUPAC name 8-Hydroxy-2,2,14,14-tetramethyl-pentadecanedioic acid. Bempedoic acid is a small molecule inhibitor of adenosine triphosphate-citrate lyase (ACL), an enzyme upstream of 3-hydroxy-3-methylglutaryl coenzyme A (HMG-CoA) reductase in the cholesterol biosynthesis pathway. Bempedoic acid is a prodrug that requires activation in liver to bempedoic acid-coenzyme A, which mediates competitive inhibition of ACL. Inhibition of ACL by bempedoic acid-coenzyme A decreases cholesterol synthesis in the liver leading to increased low-density lipoprotein receptor (LDLR) expression and LDL particle clearance from the blood. Therefore, inhibition of ACL by bempedoic acid-coenzyme A decreases low-density lipoprotein cholesterol (LDL-C) via the same pathway as HMG-CoA reductase inhibition by statins. An important differentiating feature of bempedoic acid is that, unlike statins, it does not inhibit cholesterol synthesis in skeletal muscle. The enzyme required to convert bempedoic acid to bempedoic acid-coenzyme A is not present in skeletal muscle. Therefore, bempedoic acid is not anticipated to mediate the adverse effects associated with inhibition of biological intermediates within the cholesterol biosynthesis pathway in skeletal muscle.

Bempedoic acid is a once-daily LDL-C lowering agent in phase 3 clinical trials. In phase 1 and 2 studies, bempedoic acid was efficacious in lowering LDL-C when used as monotherapy and when added to a statin and/or ezetimibe and was well tolerated in patients with statin intolerance.

WO 2004/067489 A2 (Dasseux et al.) is an international patent application directed to a class of hydroxyl compounds for use in treating high low-density lipoprotein cholesterol levels including bempedoic acid. The synthesis of bempedoic acid is described in examples 6.19 and 6.20 which is outlined below (scheme 1) as a five-step synthesis:

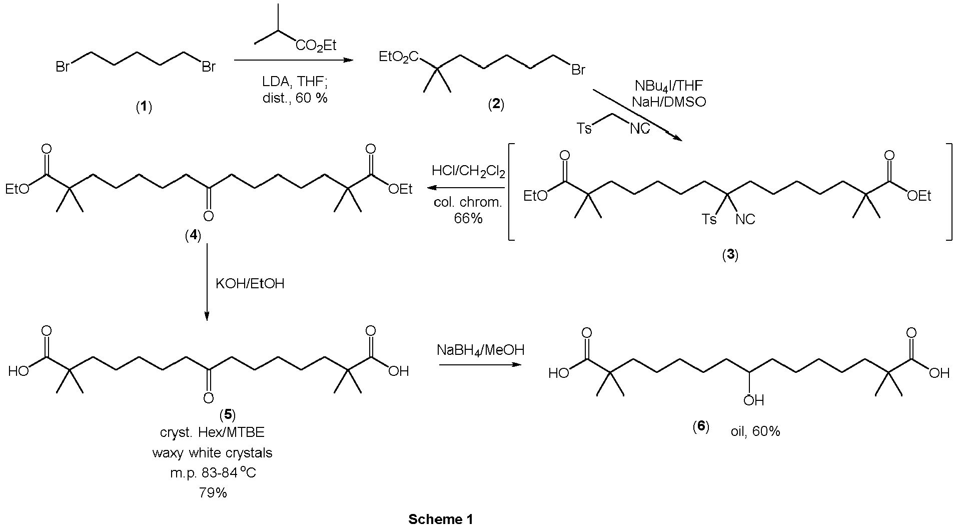

Starting from dibromopentane (1) bempedoic acid (6) can be prepared in five steps. The penultimate step is the diester hydrolysis of compound (4) to the keto-acid (5). The last step of the synthesis is the reduction of the keto-acid (5) to bempedoic acid (6) which can only be isolated as a very viscous oil in 60% yield and a purity of 83.8% as measured by HPLC.

The synthesis disclosed in the prior art has the disadvantage that it provides bempedoic acid as a viscous oil which is more difficult to handle compared to a solid. In particular, transferring the viscous oil from one vessel to another, e.g. for the production of a pharmaceutical preparation, may be difficult, as viscous oils cannot be easily transferred using pipettes. Further, solvents are more difficult to be removed from viscous oils than from solids. In particular, a longer time is generally needed. Accordingly, purification of viscous oils is more time consuming than of solids.

Finally, also for the production of solid pharmaceutical compositions it is generally desirable to have crystalline active ingredients which render the process of formations of said compositions easier, as crystalline active ingredients are generally more stable and less hygroscopic than amorphous active ingredients. Further, solid, in particular crystalline active ingredients can be mixed more easily with solid pharmaceutical excipients needed for the preparation of solid pharmaceutical compositions compared to viscous oils.

Due to the above described disadvantages in the prior art, an object of the present invention is to provide bempedoic acid in solid, in particular crystalline form. A further object of the present invention is to provide a novel synthesis of bempedoic acid which provides bempedoic as a solid, in particular crystalline form. It is a further object of the invention to provide a solid, in particular crystalline form of bempedoic acid that allows the preparation of a pharmaceutical composition wherein the solid state of the bempedoic acid is uniform. It is a further object of the invention to provide a solid, in particular crystalline form of bempedoic acid that allows the preparation of a pharmaceutical composition having predictable and reproducible dissolution properties. It is a further object of the invention to provide a solid, in particular crystalline form of bempedoic acid that enables the provision of pharmaceutical compositions having predictable and reproducible pharmacokinetic properties in patients.

### Summary of the Invention

According to the present invention, the above objectives have unexpectedly been solved as described herein.

A first aspect of the present invention is crystalline bempedoic acid of Formula I

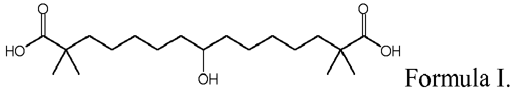

A further aspect of the present invention is a novel process of forming crystalline bempedoic acid of Formula I 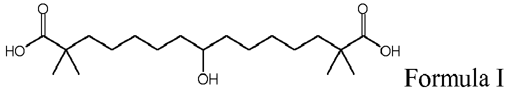 comprising the step of transforming the compound of Formula II 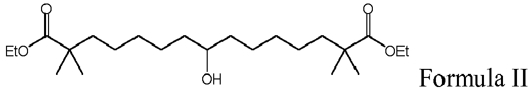 into bempedoic acid of Formula I. Bempedoic acid as obtained by the novel process of the present invention is crystalline, contrary to the process of WO 2004/067489 A2 which led to bempedoic acid as a highly viscous oil. It was unexpectedly found that by switching the order of the last two steps of the synthesis of WO 2004/067489 A2, i.e. by carrying out the ester hydrolysis as a last step, solid, in particular crystalline bempedoic acid, could be obtained in good yield.

A further aspect of the present invention is a pharmaceutical composition comprising crystalline bempedoic acid.

A yet further aspect of the present invention is a use of crystalline bempedoic acid for the preparation of a pharmaceutical formulation.

Finally, an aspect of the present invention is the use of crystalline bempedoic acid for the preparation of amorphous bempedoic acid.

### Description of the Figures

- **Figure 1:**: XRPD of crystalline bempedoic acid
- **Figure 2:**: FTIR spectrum of crystalline bempedoic acid
- **Figure 3:**: DSC curve of crystalline bempedoic acid
- **Figure 4:**: TGA curve of crystalline bempedoic acid

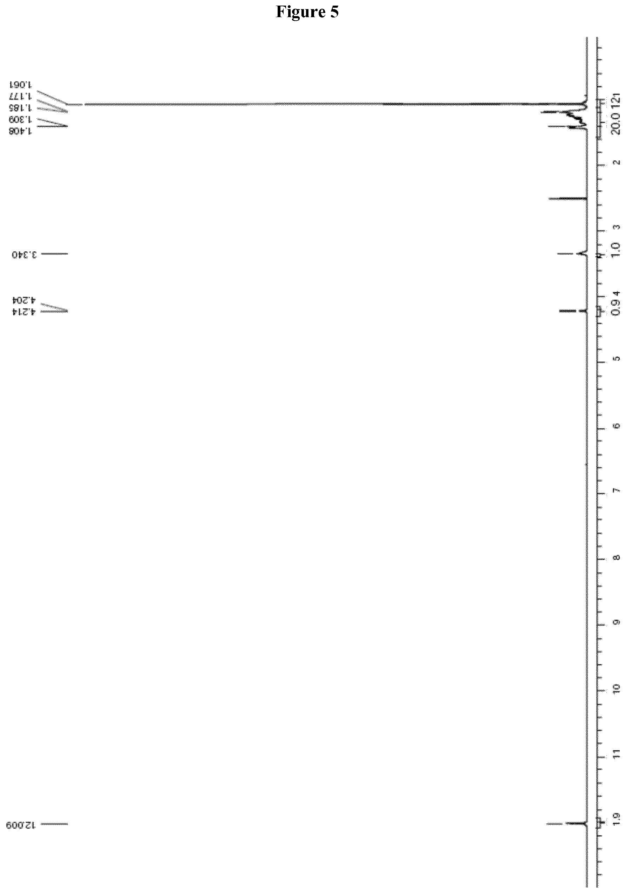

- **Figure 5:**: ¹H-NMR spectrum of bempedoic acid

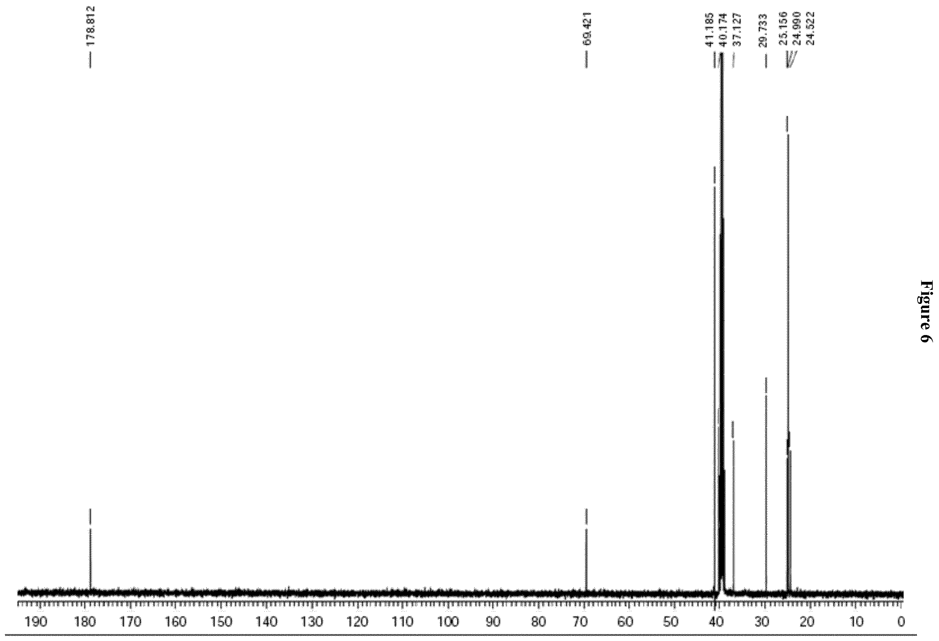

- **Figure 6:**: ¹³C-NMR spectrum of bempedoic acid

### Detailed Description of the Invention

For the avoidance of doubt, it is hereby stated that the information disclosed earlier in this specification under the heading "Background of the invention" is relevant to the invention and is to be read as part of the disclosure of the invention.

Throughout the description and claims of this specification, the singular encompasses the plural unless the context otherwise requires. In particular, where the indefinite article is used, the specification (which term encompasses both the description and the claims) is to be understood as contemplating plurality as well as singularity, unless the context requires otherwise.

All of the features disclosed in this specification (including any accompanying claims, abstract and drawings) and/or all of the steps of any process so disclosed may be combined in any combination (if not otherwise stated), except combinations where at least some of such features and/or steps are mutually exclusive. The invention is not restricted to the details of any foregoing embodiments. The invention extends to any novel feature, or any novel combination of the features disclosed in this specification (including any accompanying claims, abstract and drawings), or to any novel step, or any novel combination of the steps of any process so disclosed.

In the context of the present invention, the term "active pharmaceutical ingredient" (API) is defined as a substance used in a finished pharmaceutical dosage form (medicament) intended to furnish pharmacological activity or to otherwise have a direct effect on the diagnosis, cure, mitigation, treatment or prevention of disease, or to have direct effect in restoring, correcting or modifying physiological functions in human beings. As used herein, the active pharmaceutical ingredient can refer to its pharmaceutically acceptable salts, hydrates or solvates thereof as well as the free base/acid thereof.

"Crystalline" as used herein defines a solid material whose constituent atoms, molecules, or ions are arranged in an ordered pattern extending in all three spatial dimensions and thus exhibit a periodic arrangement over a great range. Due to the long range order of the corresponding constituents, a crystal lattice may be formed which extends in all directions. In addition, a crystalline form may be characterized in that the X-ray powder diffractogram (XRPD) exhibits a pattern of distinct characteristic peaks. Contrary to a crystalline substance, the XRPD of an amorphous or non-crystalline substance shows a pattern which due to the absence of characteristic peaks shows a more or less elevated base line. Further, a crystalline substance may be characterized by having a distinct melting point.

Crystalline bempedoic acid as provided by the present invention may be characterized by analytical methods well known in the field of the pharmaceutical industry for characterizing solids. Such methods comprise but are not limited to X-ray powder diffraction (XRPD), Fourier transform infrared (FTIR) spectroscopy, differential scanning calorimetry (DSC), thermogravimetric analysis (TGA) and gravimetric moisture sorption (GMS). Crystalline bempedoic acid may be characterized by one of the aforementioned methods or by combining two or more of them. In particular, crystalline bempedoic acid may be characterized by one of the following embodiments or by combining two or more of the following embodiments.

In a preferred embodiment crystalline bempedoic acid can be characterized by an X-ray powder diffractogram comprising diffraction peaks at 10.3,18.0,20.3 and 21.8 °2θ. These peaks may be regarded as particularly characteristic diffraction peaks for crystalline bempedoic acid. Preferably, the diffractogram comprises further peaks at 15.6, 17.3 and 17.5 °2θ. More preferably, additional peaks can be observed at 11.7, 18.7, 19.5, 22.5, 23.1, 23.5, 27.6, 30.8 and/or 34.4 °2θ.

In a further embodiment, bempedoic acid can be characterized by an X-ray powder diffractogram comprising following diffraction peaks and relative intensity:

| | | | | | | | | | |
|---|---|---|---|---|---|---|---|---|---|
| Pos. [°2θ] | 10.3 | 11.7 | 14.4 | 15.6 | 17.0 | 17.3 | 17.5 | 18.0 | 18.2 |
| Rel. Int. [%] | 59 | 9 | 4 | 17 | 11 | 40 | 99 | 100 | 33 |

| | | | | | | | | | |
|---|---|---|---|---|---|---|---|---|---|
| Pos. [°2θ] | 18.7 | 19.5 | 20.3 | 20.7 | 21.1 | 21.8 | 22.5 | 23.1 | 23.5 |
| Rel. Int. [%] | 46 | 30 | 72 | 15 | 6 | 47 | 26 | 20 | 27 |

| | | | | | | | | | |
|---|---|---|---|---|---|---|---|---|---|
| Pos. [°2θ] | 23.9 | 24.6 | 25.2 | 25.7 | 26.3 | 27.6 | 29.1 | 29.8 | 30.5 |
| Rel. Int. [%] | 10 | 6 | 4 | 4 | 5 | 11 | 8 | 6 | 6 |

| | | | | | | | |
|---|---|---|---|---|---|---|---|
| Pos. [°2θ] | | 30.8 | 31.7 | 32.8 | 34.4 | 35.0 | 36.2 |
| Rel. Int. [%] | | 9 | 5 | 4 | 9 | 3 | 8 |

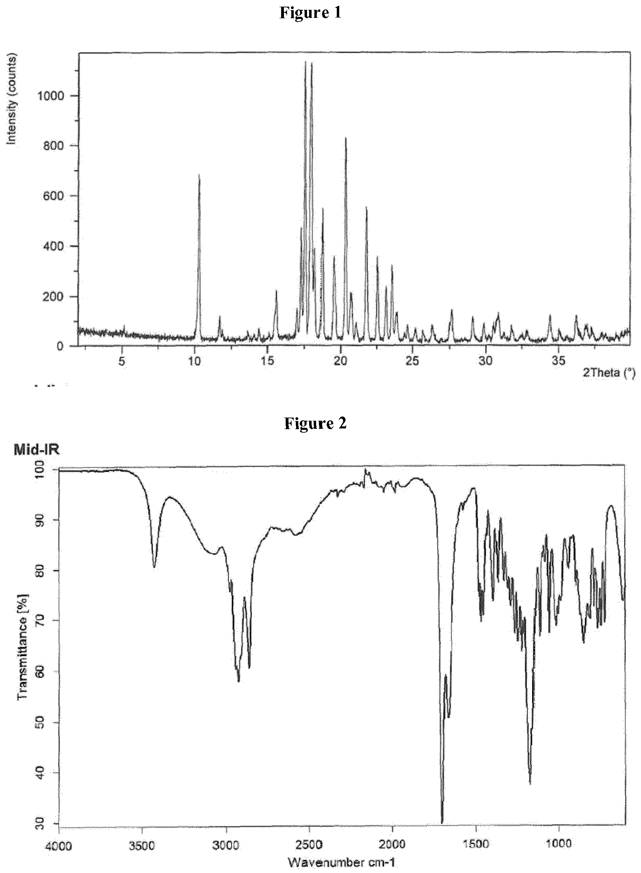

Alternatively, crystalline bempedoic acid can preferably be characterized by having a powder X-ray diffractogram essentially the same as displayed in Figure 1 of the present invention.

In the present application, the XRPD is recorded as described in the experimental section below. Further, unless indicated otherwise, XRPD peaks are reported as °2θ values with a standard error of ± 0.2 °2θ.

In a further embodiment, crystalline bempedoic acid can be preferably characterized by having a Fourier transform infrared (FTIR) spectrum comprising peaks at wavenumbers of (3424±4) cm⁻¹, (2922±4) cm⁻¹, (2856±4) cm⁻¹, (1705±4) cm⁻¹, (1667±4) cm⁻¹ and (1171±4) cm⁻¹. Further preferably, crystalline bempedoic acid can be preferably characterized by having a Fourier transform infrared spectrum comprising peaks at following wavenumbers [cm⁻¹]:

| | | | | | | | | | |
|---|---|---|---|---|---|---|---|---|---|
| 3424 | 2922 | 2856 | 1705 | 1667 | 1455 | 1392 | 1363 | 1326 | 1304 |
| 1285 | 1264 | 1242 | 1220 | 1171 | 1114 | 1082 | 1057 | 1014 | 897 |

Alternatively, crystalline bempedoic acid can preferably be characterized by having a Fourier transform infrared spectrum essentially the same as displayed in Figure 2 of the present invention. In the present application the Fourier transform infrared spectra is recorded as described in the experimental section below. Further, unless indicated otherwise, FTIR spectrum wavenumbers are reported as cm⁻¹ values with a standard error of ± 4 cm⁻¹.

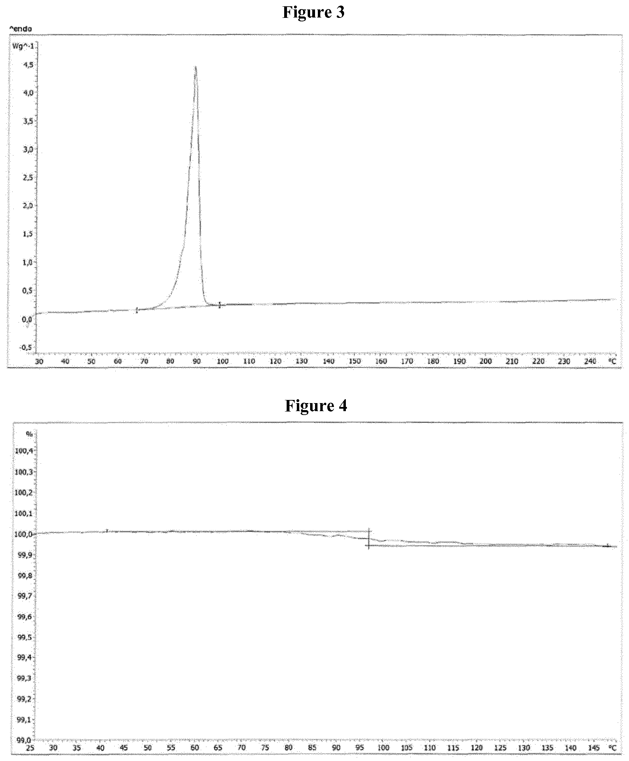

In a further embodiment, crystalline bempedoic acid can preferably have a melting point of about 85°C, as determined by the onset of the melting endotherm using DSC with a heating rate of 10°C/min. The endothermic peak is at about 89°C. In the present application the DSC is recorded as described in the experimental section below. A differential scanning calorimetric curve of bempedoic acid is shown in Figure 3.

In yet a further embodiment, crystalline bempedoic acid is characterized by showing a weight loss of about 0.1% or less based on the weight of crystalline bempedoic acid, when measured with thermogravimetric analysis. In the present application, the TGA is recorded as described in the experimental section below. The result of a thermogravimetric analysis of bempedoic acid is shown in Figure 4.

A further aspect of the present invention is a novel synthesis of solid, in particular crystalline bempedoic acid. The process of forming bempedoic acid of Formula I 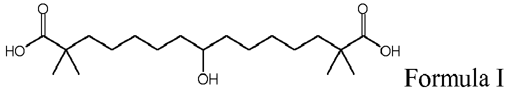 comprises the step of transforming the compound of formula II 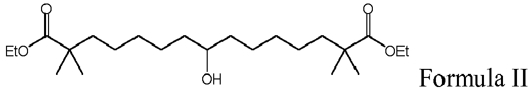 into bempedoic acid of Formula I.

In a preferred embodiment, the step of transforming the compound of Formula II into bempedoic acid of Formula I - which is a hydrolysis of a diester - is carried out in the presence of a base or an acid. It was unexpectedly found that carrying out the ester hydrolysis as the last step of the synthesis of bempedopic acid enables to obtain the product in solid, in particular crystalline form. Whilst not being bound by any theory, the inventors assume that the cause for this unexpected effect might lie in the different nature of reagents and/or impurities in the crude product.

As defined herein, a "base" is a substance that, in aqueous solution, releases hydroxide (OH⁻) ions. As defined herein, an "acid" is a substance that donates protons (H⁺).

In an embodiment of the invention, the step of transforming the compound of formula II into bempedoic acid of Formula I is carried out in the presence of an acid. The acid is not limited, as long as it can catalyse the reaction by protonating the ester carbonyl making it more electrophile. Examples of acids that can be used in the step of transforming the compound of Formula II into bempedoic acid of Formula I comprise sulphuric acid, sulphurous acid, nitric acid, nitrous acid, hydrochloric acid, sulfonic acids, phosphoric acids.

Sulfonic acids for example are preferably chosen in the group consisting of perhalogenated sulfonic acids, such as triflic acid (TFA); benzyl derivatives sulfonic acids, such as benzyl sulfonic acid (BSA), p- toluenesulfonic acid (PTSA), and dodecyl benzyl sulfonic acid (DBSA); - alkyl sulfonic acids, such as propyl sulfonic acid; cycloalkyl sulfonic acid, such as 10-camphorsulfonic acid (CSA); and alkoxyglyceryl sulfonic acids, such as lauryl glyceryl sulfonic acid.

In a preferred embodiment of the invention, the step of transforming the compound of Formula II into bempedoic acid of Formula I is carried out in the presence of a base. The base is selected from the group consisting of inorganic bases or organic amino bases, such as guanidine or pyridine. Preferably, the base is selected from inorganic bases, in particular metal hydroxides and ammonium hydroxide. Among the metal hydroxides, metal hydroxides of alkali or earth alkali metals are preferred. More preferred are alkali earth metal hydroxides LiOH, NaOH, KOH, RbOH and CsOH, even more preferred are LiOH, NaOH and KOH.

The amount of inorganic base required in the current invention should be at least stoichiometric to the amount of ester groups available for hydrolysis for complete conversion. Thus, the mole ratio of inorganic base to hydrolyzable ester groups can be 1:1 to 2:1, such as 1.01:1, 1.1:1, 1.2:1, 1.3:1, 1.4:1, 1.5:1, 1.6:1, 1.7:1, 1.8:1, 1.9:1. Preferably, the mole ratio of inorganic base to hydrolyzable ester groups is 1.01:1 to 1.5:1, such as 1.05:1, 1.1:1, 1.2:1, 1.3:1, 1.4:1.

The step of transforming the compound of Formula II into bempedoic acid of Formula I is carried out in a suitable solvent. A solvent is regarded as a substance, preferably a substance being liquid at 23°C at 1013 mbar, which is able to at least partially dissolve a solute, wherein in the present case the compound of Formula II is the solute.

In a preferred embodiment of the invention the solvent has a boiling point at 1013 mbar between 50°C and 150°C, preferably between 60°C and 120°C, more preferably between 70°C and 110°C and in particular between 75°C and 100°C.

The solvent can preferably be an alcohol, which is a compound represented by a hydroxy group (-OH) bound to a carbon atom, or a ketone, which is a compound represented by the structure R¹C(=O)R², where R¹ and R² can be a carbon-containing group, or an ether.

Examples of alcohols are methanol, ethanol, 1-propanol, 2-propanol, 2-methyl-1-propanol, 2-methyl-2-propanol, 2,2-dimethyl-1-propanol, 1-butanol, 2-butanol, 2-methyl-1-butanol, 2-methyl-2-butanol, 3-methyl-1-butanol, 3-methyl-2-butanol, 1-pentanol and 1-hexanol.

Examples of ketones are 2-pentanone, 3-pentanone, 2-hexanone, 3-hexanone and methyl isobutyl ketone.

Examples of ethers are tetrahydrofurane (THF), dioxane, dimethyl ether, diethyl ether, methyl *t*-butyl ether, diethylene glycol dimethyl ether, diisopropyl ether.

In a preferred embodiment of the invention the solvent is ethanol, 2-methyl-1-propanol, 2 butanol, 1-propanol, 1-pentanol, methyl isobutyl ketone or mixtures thereof.

In a particularly preferred embodiment the solvent is ethanol.

In an equally preferred embodiment the solvent is a mixture of an alcohol or an ether with water. Preferably, the solvent is a mixture of an alcohol with water. In one embodiment, the alcohol is mixed with water in a ratio (v/v) of 25:1, 20:1, 15:1, 10:1, 8:1, 6:1, 5:1, 4:1, 3:1, 2:1, 1:1, 1:1.5, 1:2, preferably 8:1, 6:1, 5:1, 4:1, 3:1, 2:1, 1:1, 1:1.5, more preferably 4:1, 3:1, 2:1, 1:1. As mentioned above, the most preferred alcohol is ethanol.

In an embodiment of the invention, the step of transforming the compound of Formula II into bempedoic acid of Formula I is carried out at the boiling point of the solvent. Preferably, the step of transforming the compound of Formula II into bempedoic acid of Formula I is carried out at a temperature in the range of 25 °C to 150°C, more preferably 35 °C to 120°C, even more preferably 50°C to 110°C, most preferably 65°C to 100°C.

In an embodiment of the invention, the compound of Formula II and the acid or base are reacted for a time period in the range of 1 to 24 h, preferably 2 to 12 h.

After completion of the reaction, crude bempedoic acid can be obtained after standard procedures, such as standard work-up to neutralize the reaction mixture and extraction. Bempedoic acid can then be purified using common purification steps including extraction, column chromatography, recrystallization or a mixture thereof.

In a preferred embodiment of the invention, the crude bempedoic acid is purified by recrystallization from a suitable solvent to obtain crystalline bempedoic acid as described above. Preferably, the solvent is an alcohol or an ether. Examples of alcohols are ethanol, 2-methyl-1-propanol, 2 butanol, 1-propanol, 1-pentanol, methyl isobutyl ketone or mixtures thereof.

In a preferred embodiment of the invention the solvent is an ether such as tetrahydrofurane (THF), dioxane, dimethyl ether, diethyl ether, methyl *t*-butyl ether, diethylene glycol dimethyl ether, diisopropyl ether.

In a particularly preferred embodiment the solvent is diisopropyl ether.

Recrystallisation can be carried out according to common procedures. A common procedure for the purification of a solid material by recrystallisation from a solution involves the steps:
(a) The material containing impurities is dissolved in a suitable solvent at or near the boiling point, to form a near-saturated solution.
(b) The hot solution can be filtered to remove any insoluble particles.
(c) The solution is allowed to cool so that the dissolved substance crystallises out.
(d) The crystals are separated from the mother liquor, by centrifuging or by filtering.
(e) The crystals are washed free from mother liquor with a little fresh cold solvent, then dried.

The process of the present invention further comprises the step of obtaining the compound of Formula II from the compound of Formula III

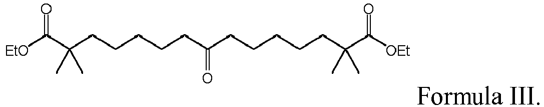

The step of obtaining the compound of Formula II from the compound of Formula III is thus a reduction of the carbonyl moiety of a keto diester to the corresponding alcohol, leaving the diester intact. While in WO 2004/067489 A2 the reduction of the keto group to the alcohol is the last step in the process of producing bempedoic acid (with the two diester groups being already hydrolysed), the present inventors have unexpectedly found out that it is advantageous to anticipate this reaction before the diester hydrolysis, since carrying out the diester hydrolysis of compound of Formula II as the last step allows to obtain bempedoic acid of Formula I in crystalline form.

In an embodiment of the invention, the step of obtaining the compound of Formula II from the compound of Formula III is carried out in the presence of a reducing agent and a solvent.

In this step, the solvent can preferably be an alcohol, an ether or a mixture thereof. Examples of alcohols are methanol, ethanol, 1-propanol, 2-propanol, 2-methyl-1-propanol, 2-methyl-2-propanol, 2,2-dimethyl-1-propanol, 1-butanol, 2-butanol, 2-methyl-1-butanol, 2-methyl-2-butanol, 3-methyl-1-butanol, 3-methyl-2-butanol, 1-pentanol and 1-hexanol.

Examples of ethers are tetrahydrofurane (THF), dioxane, dimethyl ether, diethyl ether, methyl *t*-butyl ether, diethylene glycol dimethyl ether, diisopropyl ether.

Preferably, the solvent is selected from the group consisting of methanol, ethanol, THF, diethylether and mixtures thereof. More preferably, the solvent is methanol or ethanol.

As a reducing agent, any agent which is able to reduce a keto group in the presence of two ester groups can be used. In an embodiment of the invention, the reducing agent is selected from NaBH₄, KBH₄, LiBH₄ or hydrogen. In a preferred embodiment, the reducing agent is NaBH₄. In case of hydrogen as reducing agent, the reaction is carried out in the presence of a metal catalyst such as nickel, palladium or platinum, preferably on a support material such as activated carbon.

In an embodiment of the invention the molar ratio between the compound of Formula III and the reducing agent can be in the range of 1:1 to 1:10, preferably 1:05 to 1:4.

In an embodiment of the invention, the step of obtaining the compound of Formula II from the compound of Formula III is carried out at a temperature in the range of -20 °C to 20 °C, preferably -10 °C to 10 °C, more preferably -5 °C to 5°C.

In a further embodiment of the process according to the invention, the process further comprises the step of obtaining the compound of Formula III from the compound of Formula IV

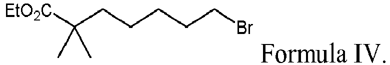

Preferably, the step of obtaining the compound of Formula III from the compound of Formula IV is carried out with Tosylmethylisocyanide and a base in a suitable solvent.

The solvent is selected from polar aprotic solvents, such as DMSO, acetone, *N,N-*dimethylformamide (DMF), acetonitrile, and THF. Preferably, the solvent is DMSO. Preferably, the base is selected from NaH, n-butyllithium, lithium diisopropylamide, LiHMDS, LiTMP, potassium *tert*-butoxide and mixtures thereof. More preferably, the base is NaH.

Optionally, a phase transfer catalyst such as tetrabutylammonium iodide can be added to the reaction mixture.

In an embodiment of the invention, the step of obtaining the compound of Formula III from the compound of Formula IV is carried out first at a temperature in the range of -20 °C to 15 °C, preferably -10 °C to 10 °C, more preferably -5 °C to 5°C, while the base is added to the reaction mixture. After the addition of the base, the reaction mixture is allowed to warm to room temperature (25°C). Preferably, the reaction mixture is then stirred at room temperature for 1 to 24 h, preferably 8 to 16 h.

In order to obtain the compound of Formula III, the reaction mixture is then preferably quenched with a sufficient amount of water and then reacted with an acid. Examples of acids comprise sulphuric acid, sulphurous acid, nitric acid, nitrous acid, hydrochloric acid, sulfonic acids, phosphoric acids. Sulfonic acids for example are those described above. Preferably, the acid is selected from sulphuric acid and hydrochloric acid.

The compound of Formula IV can then be obtained following standard procedures, such as extraction. The crude compound of Formula IV can then be purified using common purification steps including column chromatography.

As described above, bempedoic acid as obtained by the process of the present invention was surprisingly found to be crystalline, which qualifies it to be used for the preparation of pharmaceutical compositions, preferably solid pharmaceutical compositions.

Hence, a further aspect of the present invention is a pharmaceutical composition comprising crystalline bempedoic acid, preferably an effective and/or predetermined amount of crystalline bempedoic acid, and at least one pharmaceutical excipient.

In an embodiment of the invention, the pharmaceutical composition is essentially free of bempedoic acid in amorphous form, as determined by XRPD. "Essentially free" in the context of the present invention means that no amorphous form can be detected by X-ray powder diffraction (XRPD) measurement, i.e. no elevation of the base line can be observed in an XRPD measurement. While a crystalline phase usually produces a distinctive XRPD pattern comprising sharp lines/peaks, amorphous materials produce a broad background signal. Hence, in an embodiment of the invention the crystalline bempedoic acid used in the pharmaceutical composition of the present invention preferably has a phase purity of 90% or more, more preferably of 95% or more, most preferably of 98% or more, as determined via XRPD.

In an embodiment of the invention, the total amount of bempedoic acid in the pharmaceutical composition is in the range of 10 to 300 mg, such as 10, 20, 30 , 40 , 50, 60, 80, 100, 120, 140, 160, 180, 200, 220, 240, 260, 280, 300 mg. Preferably, the total amount of bempedoic acid in the pharmaceutical composition is in the range of 40 to 250 mg, such as 40, 50, 60, 80, 100, 120, 140, 150, 160, 170, 180, 190, 200, 220, 240, 250 mg. More preferably, the total amount of bempedoic acid in the pharmaceutical composition is in the range of 120 to 200 mg, such as 120, 130, 140, 150, 160, 170, 180, 190, 200 mg. In a particularly preferred embodiment, the total amount of bempedoic acid in the pharmaceutical composition is in the range of 160 to 200 mg, in particular 180 mg.

In an embodiment, bempedoic acid can be present in the pharmaceutical composition of the present invention in an amount of 10 to 99 wt%, preferably 30 to 80 wt%, more preferably 40 to 70 wt%, based on the total weight of the pharmaceutical composition.

In addition to crystalline bempedoic acid, the pharmaceutical composition of the invention may contain pharmaceutically acceptable salts and/or solvates of bempedoic acid. Examples of salts include hydrochloride, sulfate, bisulfate, tatrate, nitrate, citrate, bitratrate, phosphate, malate, maleate, hydrobromide, hydrojodide, fumarate, succinate, or mixtures thereof. If bempedoic acid is present in the form of a salt, the amount of the corresponding acid/base has to be added accordingly. Examples of solvates include water (i.e. a hydrate), DMF, diethyl ether, ethanol and methanol.

The pharmaceutical composition comprising crystalline bempedoic acid may be administered to a subject by multiple administration routes, including but not limited to, oral (non-limiting examples for suitable dosage forms include solid dosage forms, like tablets, powders, capsules, suppositories, sachets, troches and lozenges, and liquid dosage forms), parenteral (non-limiting examples: intravenous, subcutaneous, intramuscular), intranasal, buccal, topical, rectal, or transdermal administration routes.

Liquid pharmaceutical compositions preferably include a liquid carrier, such as water, animal or vegetable oils, mineral oils or synthetic oils. They preferably also include a buffer, a stabilizer or other pharmaceutically acceptable excipients, or mixtures thereof. Liquid pharmaceutical compositions may also contain physiological saline solution, dextrose or other saccharide solution or glycols such as ethylene glycol, propylene glycol or polyethylene glycol.

Preferably, the pharmaceutical composition of the present invention is a solid oral dosage form. The oral dosage form can be preferably provided as tablets, powders, capsules, suppositories, sachets, troches and lozenges, more preferably as a capsule or tablet. In a preferred embodiment the oral dosage form is a tablet.

More details of oral solid dosage forms are described in subsequent embodiments.

The pharmaceutical composition of the present invention comprises one or more pharmaceutical excipient(s). Suitable pharmaceutical excipients are for example disclosed in "Lexikon der Hilfsstoffe für Pharmazie, Kosmetik und angrenzende Gebiete", published by H.P. Fielder, 4th Edition, and "Handbook of Pharmaceutical Excipients", 3rd Edition, published by A.H. Kibbe, American Pharmaceutical Association, Washington, USA, and Pharmaceutical Press, London. Generally, there are no specific restrictions concerning the chemical nature of these excipients provided that the excipient(s) comprised in the pharmaceutical composition is/are pharmaceutically acceptable. A pharmaceutically acceptable excipient is an excipient which is relatively non-toxic and innocuous to a patient at concentrations consistent with the effective activity of the bempedoic acid so that any side effects ascribable to the excipient do not vitiate the beneficial effects of bempedoic acid.

Therefore, according to the present invention, pharmaceutical excipients are for example fillers, binders, disintegrants, lubricants, glidants, coating materials, sweeteners, flavoring agents, and coloring agents such as for example pigments. Other excipients known in the field of pharmaceutical compositions/oral dosage forms may also be used, such as surfactants. Preferably, the combined amount of excipients comprises 1 to 90% by weight of the formulation or dose.

Fillers can be used to increase the bulk volume and weight of a low-dose drug to a limit at which a pharmaceutical dosage form can be formed. Fillers may fulfil several requirements, such as being chemically inert, non-hygroscopic compatible with the other components of the formulation, non-hygroscopic, relatively cheap, compactible, and preferably tasteless or pleasant tasting.

In a preferred embodiment the filler is referred to as a single filler or to a mixture of several fillers.

Examples of fillers according to the present invention include, but are not limited to, magnesium carbonate, magnesium alumino silicates, calcium carbonate, calcium phosphate, calcium hydrogen phosphate, calcium dihydrogen phosphate, a sugar alcohol, such as erythritol, isomaltose, mannitol, maltitol, sorbitol, threitol and xylitol, a monosaccharide, such as arabinose, galactose, glucose, mannose and xylose, a disaccharide, such as isomaltose, lactose (such as anhydrous lactose or lactose monohydrate form), maltose, sucrose, an oligosaccharide, such as cyclodextrin, maltodextrin and raffinose, a polysaccharide, such as cellulose (e.g. microcrystalline cellulose, silicated microcrystalline cellulose), glycogen and starch. In a preferred embodiment, the filler can preferably be selected from microcrystalline cellulose, lactose, mannitol and starch, more preferably from lactose and microcrystalline cellulose.

Fillers can preferably be present in an amount of 1 to 90 wt%, more preferably from 10-60 wt%, in particular from 20-55 wt%, based on the total weight of the oral dosage form.

Binders should be capable of ensuring that granules or tablets can be formed with the required mechanical strength and of giving volume to low active dose tablets. Suitable binders according to the present invention include, but are not limited to, hydroxypropyl cellulose, hypromellose (hydroxypropyl methylcellulose, HPMC), acacia, alginic acid, carboxymethyl cellulose, ethyl cellulose, methylcellulose, hydroxyethyl cellulose, ethylhydroxyethyl cellulose, polyvinyl alcohol, polyacrylates, carboxymethyl cellulose calcium, carboxymethyl cellulose sodium, compressible sugar, ethyl cellulose, gelatin, liquid glucose, methylcellulose, polyvinyl pyrrolidone and pregelatinized starch. In an alternative preferred embodiment the oral dosage form of the present invention does not comprise a binder.

Binders can be present in an amount of 0 to 40 wt%, preferably in an amount of 3 to 30 wt%, in particular 4 to 20 wt%, based on the total weight of the oral dosage form.

Disintegrants are compounds which enhance the ability of the dosage form, preferably the ability of the tablet, to break into smaller fragments when in contact with a liquid, preferably water. Suitable disintegrants according to the present invention include, but are not limited to, carboxymethyl cellulose calcium, carboxymethyl cellulose sodium, croscarmellose (crosslinked carboxymethyl cellulose) sodium (e.g. Ac-Di-Sol®, Primellose®), cross-linked polyvinylpyrrolidone, crospovidone (cross-linked povidone, a synthetic cross-linked homopolymer of N-vinyl-2-pyrrolidone), alginic acid, microcrystalline cellulose (such as refined wood pulp derived from alpha cellulose), hydroxypropyl cellulose (HPC), low substituted hydroxypropyl cellulose, polacrillin potassium, sodium alginate, sodium starch glycolate, partially hydrolysed starch, sodium carboxymethyl starch, and starch.

Disintegrants can be present for example in an amount of 0 to 10 wt%, preferably in an amount of 0.25 to 8.5 wt% in particular in an amount of 0.5 to 5 wt% based on the total weight of the oral dosage form.

Lubricants generally can be regarded as substances which are suitable to reduce friction, such as static friction, sliding friction and rolling friction. In particular, lubricants reduce the shearing forces occurring on the borderline between tablet and mould, especially the sliding friction found during tablet pressing between the punch moving up and down in the die and the die wall on the one hand and between the edge of the tablet and the die wall on the other hand. Suitable lubricants according to the present invention include, but are not limited to, calcium stearate, magnesium stearate, mineral oil, stearic acid, fumaric acid, adipic acid, sodium stearyl fumarate, zinc stearate and polyethylene glycol, in particular magnesium stearate.

Lubricants can be present for example in an amount of 0 to 5 wt%, preferably 0.5-4 wt%, in particular 0.75-3 wt%, based on the total weight of the oral dosage form.

Glidants can be used to improve the flowability. Suitable glidants are for example colloidal silicon dioxide (e.g. Aerosil®), talc or mixtures thereof. Preferably, glidants can be present in an amount of 0 to 5 wt%, preferably 0.5-4 wt% glidant, in particular 0.75-3 wt% glidant, based on the total weight of the oral dosage form.

The coating materials comprise coating materials known in the art. An example of coating material for forming a coating film is Opadry II.

Sweeteners are added to make the ingredients more palatable. Suitable sweeteners are, for example, sorbitol, dextrose, sucrose, saccharin, aspartame, acesulfame-K and sucralose. Preferably, sweeteners can be present in an amount of 0 to up to 10 wt.%, more preferably of 0.2 to 8 wt.%, in particular of 0.5 to 6 wt.% based on the total weight of the dosage form.

Flavouring agents can be used to mask unpleasant taste of the active ingredients and increase the probability that the patient will complete a course of medication. The U.S. Code of Federal Regulations describes a natural flavorant as: an essential oil, oleoresin, essence or extractive, protein hydrolysate, distillate or any product of roasting, heating or enzymolysis which contains the flavoring constituents derived from a spice, fruit or fruit juice, vegetable or vegetable juice, edible yeast, herb, bark, bud, root, leaf or similar plant material, dairy products or fermentation products thereof whose significant function in food is flavoring rather than nutritional.

Artificial flavors include any substance the function of which is to impart flavor which is not derived from a spice, fruit or fruit juice, vegetable or vegetable juice, edible yeast, herb, bark, bud, root, leaf or similar plant material, meat, fish, poultry, eggs, dairy products or fermentation products thereof.

Preferably, flavours can be present in an amount of 0 to up to 5 wt.%, more preferably of 0.2 to 4 wt.%, in particular of 0.3 to 3 wt.% based on the total weight of the dosage form.

Suitable coloring agents according to the present invention include, but are not limited to, pigments, inorganic pigments, FD&C Red No. 3, FD&C Red No. 20, FD&C Yellow No. 6, FD&C Blue No. 2, D&C Green No. 5, D&C Orange No. 5, D&C Red No. 8, caramel, ferric oxide red, ferric oxide yellow and titanium dioxide.

Surfactants (or wetting agents) can increase the solubility of pharmaceutical composition or bempedoic acid. Suitable surfactants according to the present invention include, but are not limited to, suitable anionic surfactants such as sodium dodecyl sulphate (sodium lauryl sulphate); docusate sodium; Include non-limiting examples of suitable cationic surfactants such as cetrimide, benzethonium chloride, cetylpyridinium chloride and lauric acid; Include non-limiting examples of suitable nonionic surfactants such as polyoxyethylene castor oil derivates, e.g. polyoxyethyleneglycerol triricinoleate or polyoxyl castor oil (Cremophor EL; BASF Corp.) or polyoxyethyleneglycerol oxystearate such as polyethylenglycol 40 hydrogenated castor oil (Cremophor RH 40, also known as polyoxyl 40 hydrogenated castor oil or macrogolglycerol hydroxystearate) or polyethylenglycol 60 hydrogenated castor oil (Cremophor RH 60); or a mono fatty acid ester of polyoxyethylene sorbitan, such as a mono fatty acid ester of polyoxyethylene (20) sorbitan, e.g. polyoxyethylene (20) sorbitan monooleate (Tween 80), polyoxyethylene (20) sorbitan monostearate (Tween 60), polyoxyethylene (20) sorbitan monopalmitate (Tween 40), or polyoxyethylene (20) sorbitan monolaurate (Tween 20).

Surfactants can be present in an amount of 0 to 40 wt%, preferably in an amount of 0.1 to 15 wt%, in particular 0.2-5 wt%; based on the total weight of the oral dosage form.

The skilled person will appreciate that depending on formulation context and concentration a particular excipient can fulfill various and sometimes even different functions. For example, microcrystalline cellulose is a particularly hydrolyzed cellulose, which can be used as a filler, binder and/or disintegrating material in tablet production, dependent on formulation context and concentration. Reference is made to the literature on pharmaceutical excipients and pharmaceutical formulation, such as Fiedler - Encyclopedia of Excipients for Pharmaceuticals, Cosmetics and Related Areas, Wissenschaftliche Verlags¬gesellschaft Stuttgart, 2013, Bauer, Fromming and Führer, "Lehrbuch der Pharmazeutischen Technologie", Wissenschaftliche Verlagsgesellschaft Stuttgart, 9. Auflage (2012) or, with a particular focus on tablet production, Augsburger and Stephen, Pharmaceutical Dosage Forms: Tablets, Third Edition, Volume 2, Informa Healthcare (2008). The skilled person will therefore appreciate that terms like "disintegrant", "binder", "lubricant", "filler", "plasticizer", "surfactant", "wetting agent", "film-forming agent", "coating material", "sweetener", "flavoring agent" and "coloring agent" are primarily functional definitions and that the structural characterization provided above are given so as to more easily allow identification of suitable excipients. In an embodiment of the invention, the pharmaceutical composition of the present invention does not comprise an enteric release material.

Generally, an enteric release material can be regarded as a material which, when included in the oral dosage form, ensures the passage of the active pharmaceutical ingredient without substantial dissolution in the stomach. In other words, an enteric release material can be regarded as a material which forms a barrier to prevent the dissolution of the active pharmaceutical ingredient already under gastric conditions; i.e. the enteric release material can protect the active pharmaceutical ingredient form the acidity of the stomach.

Preferably, an enteric coating material or an enteric release material can be regarded as material which is substantially insoluble at a pH value of 5.5 or lower and/or which is substantially soluble at a pH value of 6.5 or higher.

Enteric release materials are for example methylcellulose, ethylcellulose, hydroxypropyl cellulose, hydroxypropyl methyl cellulose phthalate, hydroxypropyl methyl cellulose succinate, cellulose acetate phthalate, cellulose acetate succinate, cellulose trimellitate, alkyl (meth)acrylate-(meth)acrylate copolymers, carnauba wax, xanthan, gum, gelatin, chitosan, carrageenan, alginates.

In a preferred embodiment of the invention the pharmaceutical composition of the present invention is free of an enteric coating. In is particularly preferred that the pharmaceutical composition is free of an enteric coating comprising one or more of methylcellulose, ethylcellulose, hydroxypropyl cellulose, hydroxypropyl methyl cellulose phthalate, hydroxypropyl methyl cellulose succinate, cellulose acetate phthalate, cellulose acetate succinate, cellulose trimellitate and alkyl (meth)acrylate-(meth)acrylate copolymers.

In a further embodiment of the invention, the pharmaceutical composition comprises an enteric release coating.

In a preferred embodiment, the solid dosage form according to the invention provides an immediate release of bempedoic acid. This means that the release profile of the dosage forms of the invention according to USP app. II (paddle, 900 ml, 0.1 M HCl, 50 rpm, 37°C) after 15 minutes preferably indicates a content release of the active pharmaceutical ingredient of 70% to 100%, preferably more than 75%, especially more than 80%.

In a further preferred embodiment, the solid dosage form according to the invention provides a controlled release of bempedoic acid. "Controlled release" as used herein means that pharmaceutically active substances are released from a medicament over a longer period of time than they are from known formulations for immediate release.

In an embodiment, the release takes place over a time period of two to twenty four hours, of two to twenty hours, especially preferred over a time period of two to sixteen hours or two to twelve hours, with the specifications satisfying the legal and regulating requirements. This means that the release profile of the dosage forms of the invention according to USP app. II (paddle, 900 ml, 0.1 M HCl, 50 rpm, 37°C) after 15 minutes preferably indicates a content release of the active pharmaceutical ingredient of 1% to 40%, preferably 5% to 35%, more preferably between 10% and 30% and even more preferably between 15% and 25% of bempedoic acid.

Another preferred embodiment of the invention relates to preparations that release between 25% to 65%, preferably between 30% to 60%, more preferably between 35% to 55% and even more preferably between 40% to 50% of bempedoic acid after 1 hour.

Yet another preferred embodiment of the invention relates to preparations that release between 40% to 80%, preferably between 45% to 75%, more preferably between 45% to 70% and even more preferably between 45% to 50%, 50% to 55%, 55% to 60%, 60% to 65% or 65% to 70% of bempedoic acid after 2 hours.

One preferred embodiment of the invention relates to preparations that release 70% to 100%, preferably between 75% to 95%, more preferably between 80% to 95%, and even more preferably between 80% and 90% of bempedoic acid after 4 hours.

One preferred embodiment of the invention also relates to preparations that release between 70% to 100%, preferably between 75% to 100%, more preferably between 80% to 95% and even more preferably between 80% to 85%, between 85% to 90% or between 90% to 95% of bempedoic acid after 7 hours.

Yet another preferred embodiment of the invention relates to preparations that release between 85% to 100%, preferably between 90% to 100%, more preferably between 95% to 100% and even more preferably approximately 95% or 100% of bempedoic acid after 12 hours.

In a preferred embodiment the pharmaceutical composition is an oral dosage form comprising:
- 10-99 wt% bempedoic acid,
- 1-90 wt% filler,
- 0-40 wt%, binder,
- 0-10 wt% disintegrant,
- 0-5 wt% lubricant,
- 0-5 wt% glidant,
- 0-10 wt% sweetener,
- 0-5 wt% flavouring agent,
- 0-40 wt% surfactant,
wherein the wt.% are based on the total weight of the oral dosage form.

In a preferred embodiment the pharmaceutical composition is an oral dosage form comprising:
- 10-99 wt%, preferably 30-80 wt%, in particular 40-70 wt% bempedoic acid,
- 1-90 wt%, preferably 10-60 wt%, in particular from 20-55 wt% filler,
- 0-40 wt%, preferably 3-30 wt%, in particular 4-20 wt% binder,
- 0-10 wt%, preferably 0.25-8.5 wt%, in particular 0.5-5 wt% disintegrant
- 0-5 wt%, preferably 0.5-4 wt%, in particular 0.75-3 wt% lubricant,
- 0-5 wt%, preferably 0.5-4 wt%, in particular 0.75-3 wt% glidant,
- 0-10 wt%, preferably 0.2-8 wt%, in particular 0.5-6 wt% sweetener,
- 0-5 wt%, preferably 0.2-4 wt%, in particular 0.3-3 wt% flavouring agent,
- 0-40 wt%, preferably 0.1-15 wt%, in particular 0.2-5 wt% surfactant,
wherein the wt.% are based on the total weight of the oral dosage form.

In a preferred embodiment the pharmaceutical composition is an oral dosage form comprising:
- 10-99 wt% bempedoic acid,
- 1-90 wt% microcrystalline cellulose, glucose, lactose, mannitol, starch or mixtures thereof
- 0-40 wt% gelatine, polyvinylpyrrolidone, hydroxypropyl methylcellulose, hydroxypropyl cellulose or mixtures thereof
- 0-10 wt% croscarmellose sodium, cross-linked polyvinylpyrrolidone, crospovidone, sodium starch glycolate,
- 0-5 wt% stearic acid, sodium stearyl fumarate, magnesium stearate, or mixtures thereof,
- 0-5 wt% talc and/or colloidal silicon dioxide,
wherein the wt.% are based on the total weight of the oral dosage form.

In an embodiment, the oral dosage form can preferably have a weight of 200 to 1500 mg, preferably 300 to 1000 mg. The weight of the oral dosage form enables an administration by a simple dosage regime. The amount of 300-1000 mg can preferably be administered all at once; i.e. for example by one single tablet or one capsule.

In case that the oral dosage form is a tablet, the tablet can be coated or uncoated, preferably coated, more preferably film-coated, in particular film-coated with a film coating that does not affect the release of the active agent(s).

For this purpose, the methods of film-coating dosage forms such as tablets as standard in the state of the art may be employed. The above-mentioned amounts of bempedoic acid and excipients relate to the uncoated dosage form.

Typical film-coatings are preferably formed using polymers such as modified celluloses, polyvinyl acetate phthalate, polyvinylpyrrolidone, polymethacrylates and/or shellack.

The preferred coating may comprise a film-forming agent and one or more of the following: lubricant, surfactant, glidant, pigment and water.

In a preferred embodiment the film can have a thickness of 2 µm to 150 µm, preferably 10 to 100 µm, more preferably 20 to 60 µm.

In a further embodiment, the pharmaceutical composition and/or oral dosage form may comprise one or more additional active ingredient(s) in addition to bempedoic acid. Hence, the present invention also relates to fixed dose compositions of bempedoic acid with one or more active ingredient(s). In an embodiment, the one or more additional active ingredient(s) is selected from the group consisting of a statin and ezetimibe. Preferably, the pharmaceutical composition may comprise one statin and ezetimibe as the only active ingredients in addition to bempedoic acid (combination of three APIs). Further preferably, the pharmaceutical composition may comprise one statin as the only active ingredient in addition to bempedoic acid (combination of two APIs). Yet further preferably, the pharmaceutical composition may comprise ezetimibe as the only active ingredient in addition to bempedoic acid (combination of two APIs).

Statins are used primarily as a lipid-lowering agent and for prevention of events associated with cardiovascular disease. Statins work by inhibiting HMG-CoA reductase (3-hydroxy-3-methyl-glutaryl-coenzyme A reductase), an enzyme found in liver tissue that plays a key role in production of cholesterol in the body.

In an embodiment, the pharmaceutical composition comprises one or more statins as defined by the fixed dosages of atorvastatin (10 mg or 20 mg), simvastatin (5 mg, 10 mg, or 20 mg), rosuvastatin (5 mg or 10 mg) and/or pravastatin (10 mg, 20 mg, or 40 mg). In a further embodiment, the pharmaceutical composition included one or more statins as defined by the fixed dosages of Table 1 below:

**Table 1**

| **High Intensity Statin** | **Moderate Intensity Statin** | **Low Intensity Statin** |
|---|---|---|
| Atorvastatin 40-80 mg | Atorvastatin 10-20 mg | Simvastatin 10 mg |
| Rosuvastatin 20-40 mg | Rosuvastatin 5-10 mg | Pravastatin 10-20 mg |
| Simvastatin 80 mg | Simvastatin 20-40 mg | Lovastatin 20 mg |
| | Pravastatin 40-80 mg | Fluvastatin XL 20-40 mg |
| | Lovastatin 40 mg | Pitavastatin 1 mg |
| | Fluvastatin XL 80 mg | |
| | Pitavastatin 2-4 mg | |

Atorvastatin is marketed under the trade name Lipitor®, among others, and is a compound having following chemical structure

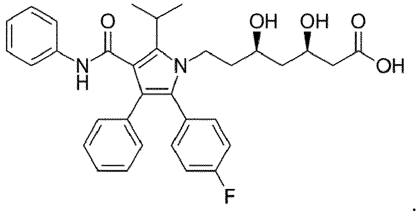

Rosuvastatin is marketed under the tradename Crestor® and has the following chemical structure

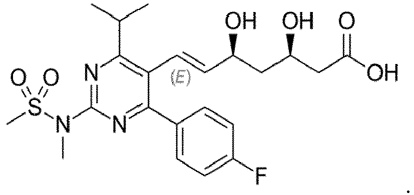

Simvastatin, marketed under the trade name Zocor® among others, has the following chemical structure

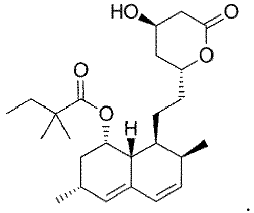

Pravastatin is marketed as Pravachol® or Selektine®, and is a compound having following chemical structure

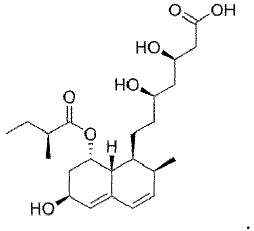

Lovastatin is marketed as Mevacor®, and is a compound having following chemical structure

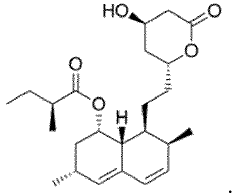

Fluvastatin is marketed as Lescol®, Canef®, Vastin®, and has the following chemical structure

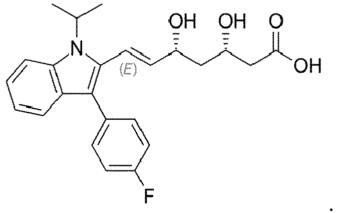

Pitavastatin is marketed as Livazo ®, and is a compound having following chemical structure

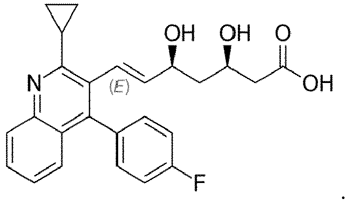

In a further embodiment, the pharmaceutical composition of the present inventions comprises ezetimibe, preferably in an absolute amount of in the range of 5 to 50 mg, more preferably 5 mg, 10 mg, 20 mg or 30 mg.

Ezetimibe is a drug that lowers plasma cholesterol levels. It acts by decreasing cholesterol absorption in the small intestine. It may be used alone (marketed as Zetia® or Ezetrol®), when other cholesterol-lowering medications are not tolerated, or together with statins (e.g., ezetimibe/simvastatin, marketed as Vytorin® or Inegy®) when statins alone do not control cholesterol. Ezetimibe is recommended as second line therapy for those intolerant of statins or unable to achieve target low-density lipoprotein (LDL) cholesterol levels on statins alone by several major medical group practice guidelines, including the American College of Cardiology. Ezetimibe is a compound of following chemical structure

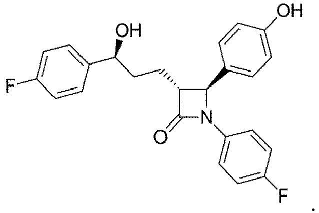

An additional aspect of the present invention is the use of the crystalline bempedoic acid for the preparation of a pharmaceutical composition.

According to the invention, the pharmaceutical composition of the invention can be prepared by a process comprising mixing crystalline bempedoic acid and optionally at least one further active ingredient together with at least one pharmaceutical excipient.

Hence, a further subject of the present invention is a method for preparing pharmaceutical composition according to the invention, in particular a tablet, comprising the steps of
(i) providing (A) bempedoic acid and (B) one or more pharmaceutical excipient(s),
(ii) optionally granulating the mixture from step (i),
(iii) compressing the mixture from step (i) or the granules from step (ii) and optionally further pharmaceutical excipients to a tablet or
   filling the mixture from step (i) or the granules from step (ii) and optionally further pharmaceutical excipients into to a capsule,
(iv) optionally coating the tablet from step (iii) with a non-enteric coating.

As far as (A) bempedoic acid and (B) one or more pharmaceutical excipient(s) are concerned for the present method, the same applies as explained above.

In step (i) bempedoic acid (A) and one or more pharmaceutical excipients (B) are provided.

Preferably, bempedoic acid (A) and (B) one or more further excipient(s) are sieved. Further, bempedoic acid (A) and (B) one or more further excipient(s) can preferably be blended in order to provide a composition having a homogenous distribution of bempedoic acid (A) and (B) one or more further excipient(s). Blending can be carried out with conventional mixing devices, e.g. in a free-fall mixer. Blending can be carried out e.g. for 1 minute to 30 minutes, preferably for 2 minutes to less than 10 minutes.

It is further preferred that the blend of bempedoic acid (A) and (B) one or more further excipient(s) can be sieved, preferably with a sieve having a mesh size of 25 to 1000 µm, preferably 50 to 800 µm, especially 100 to 600 µm.

In optional step (ii) the mixture from step (i) and one or more further excipient(s) can be granulated. It is preferred that the method of the present invention comprises step (ii). In an embodiment step (ii) comprises dry-granulating or wet-granulating the mixture of step (i).

"Dry" is usually understood to mean that the step is carried out in the absence of a liquid, in particular in the absence of water. "Granulating" is generally understood to mean the formation of relatively coarse or granular aggregate material as a powder by assembling and/or aggregating finer powder particles (agglomerate formation or build-up granulation) and/or the formation of finer granules by breaking up coarser aggregates (disintegration or break-down granulation). Dry granulation can preferably be carried out by using pressure or temperature. In a preferred embodiment of the invention, step (ii) of granulating, preferably dry-granulating, the mixture from step (i) can be performed for example by "slugging" using a large heavy-duty rotary press and breaking up the slugs into granules with a hammer mill or by roller compaction using for example roller compactors by Powtec or Alexanderwerk. The granules are then optionally screened.

"Wet" is usually understood to mean that the step is carried out in the presence of a granulation liquid, for example water, alcohols, such as isopropanol and 1-propanol, and mixtures thereof. A preferred granulation liquid is water. The granulation liquid is needed for agglomeration because it forms physical or mechanical bonds between powder particles. The bonds may consist of a binder which is provided with the liquid. The binder solidifies and forms bridges between individual powder particles as the solvent of the granulation liquid evaporates. Suitable binders for wet granulation are for example hydroxypropyl cellulose, hydroxypropyl methylcellulose or polyvinylpyrrolidone. Alternatively, the liquid may be free of binders but capable of at least partially dissolving at least one of the powder constituents. The wet granulation process can be carried out with common types of equipment which may be used for medium- and large-scale production including mixers, high-shear mixers, fluid-bed granulators, and rotary granulators. In one embodiment, the process of the invention comprises the addition of the granulation liquid to the powder from which the granules are to be formed by spraying the liquid through a nozzle onto the powder. Several types of granulation machines are equipped to allow this method, including most rotary granulators, high-shear mixers, and top-spray fluid-bed granulators. In a preferred embodiment, the granules formed in step (ii) can be dried immediately after their formation. If the granulation equipment is appropriate for this purpose, the drying step can be carried out without interruption or product transfer. An example for such type of equipment is the fluid-bed granulator. The drying of the granules can be carried out at a temperature in the range of 20-150°C, preferably 40 to 100°C, more preferably 45 to 75°C.

In step (iii) the mixture of step (i) or the granules of step (ii) and optionally further pharmaceuticals excipients can be compressed to a tablet. As far as the further pharmaceutical excipients in step (iii) are concerned the same as described above with regard to (B) one or more pharmaceutical excipient(s) applies. In case (B) one or more pharmaceutical excipient(s) is compressed with the mixture of step (i) or the granules of step (ii), blending the mixture is carried out before compression, as explained above. Compressing the mixture of step (i) or the granules from step (ii) into a tablet can preferably be carried out by compressing said formulation on a rotary press. The main compression force can range from 1 to 100 kN, preferably from 3 to 70 kN. The resulting tablets can have a hardness of 30 to 400 N, more preferably of 50 to 250 N, particularly preferably of 30 to 180 N, more preferably 40 to 150 N, wherein the hardness can be measured according to Ph.Eur. 6.0, Chapter 2.9.8.

Alternatively in step (iii) the mixture of step (i) or the granules of step (ii) and optionally further pharmaceuticals excipients can be filled into a capsule, preferably a hard gelatine capsule. For filling the mixture of step (i) or the granules of step (ii) into capsules dependent dosing systems (for example an auger) or preferably independent dosing systems (for example MG2, Matic (IMA)) can be used.

In a preferred embodiment steps (i), (ii) and (iii) can be performed under standard conditions, i.e. no specific care has to be taken with regard to humidity. In particular, these steps can be performed at a temperature from 0° to 30°C, preferably from 10 to 25°C. Further, said process is preferably performed at the humidity of 30 to 70%RH. The same conditions can be chosen for optional step (iv) as described below.

Further, the pharmaceutical composition of the invention preferably has a content uniformity, i.e. a content of active agent(s) which lies within the concentration of 90 to 110%, preferably 95 to 105%, especially preferred from 98 to 102% of the average content of the active agent(s). The "content uniformity" is determined with a test in accordance with Ph. Eur., 6.0, Chapter 2.9.6. According to that test, the content of the active agent of each individual tablet out of 20 tablets must lie between 90 and 110%, preferably between 95 and 105%, especially between 98 and 102% of the average content of the active agent(s). Therefore, the content of the active agent in each tablet of the invention differs from the average content of the active agent by at most 10%, preferably at most 5% and especially at most 2%.

In addition, the resulting pharmaceutical composition preferably has a friability of less than 5%, particularly preferably less than 2%, especially less than 1%. The friability is determined in accordance with Ph. Eur., 6.0, Chapter 2.9.7. The friability of tablets generally refers to tablets without coating.

In an optional step (iv) the tablets from step (iii) can preferably be film coated, wherein it is preferred that a coating not affecting the release of the active pharmaceutical ingredient. Preferably a film coating such as Opadry II can be used.

In case the pharmaceutical composition comprises one or more additional active pharmaceutical ingredient(s) as described above, these can be added to step (i) to bempedoic acid (A) and (B) one or more pharmaceutical excipient(s), blended and optionally granulated together.

In an alternative embodiment, one or more additional active pharmaceutical ingredient(s) is blended and optionally granulated together with one or more pharmaceutical excipient(s) and added to bempedoic acid in step (iii). That is, steps (i) and (ii) as described above for bempedoic acid can be carried out analogously with one or more additional active pharmaceutical ingredient(s). As a consequence, step (iii) can be modified to step (iiia):
(iiia) compressing the mixture from step (i) or the granules from step (ii) and a mixture of one or more additional active pharmaceutical ingredient(s) or granules containing one or more additional active pharmaceutical ingredient(s), and optionally further pharmaceutical excipients to a tablet or filling the mixture from step (i) or the granules from step (ii) and a mixture of one or more additional active pharmaceutical ingredient(s) or granules containing one or more additional active pharmaceutical ingredient(s), and optionally further pharmaceutical excipients into to a capsule.

Before compression or filling the capsules, the mixtures or granules containing bempedoic acid and the mixtures or granules containing one or more additional active pharmaceutical ingredient(s) are preferably blended, as described above.

In a further embodiment, the mixtures or granules containing bempedoic acid and the mixtures or granules containing one or more additional active pharmaceutical ingredient(s) are not blended, but compressed separately, forming a bilayer tablet. That is, bempedoic acid is in a first layer and the one or more additional active pharmaceutical ingredient(s) is in a second layer. In this embodiment, step (iii) is amended to step (iiib):
(iiib) compressing the mixture from step (i) or the granules from step (ii) and optionally further pharmaceutical excipients to a first layer, and compressing a mixture containing one or more additional active pharmaceutical ingredients or granules containing one or more additional active pharmaceutical ingredients to a second layer, thus obtaining a bilayer tablet.

In a further aspect of the present invention crystalline bempedoic acid or the pharmaceutical compositions comprising crystalline bempedoic acid can be used for medical treatment of a disease.

"Treating" a disease or disorder in a subject as used herein refers 1) inhibiting the disease or disorder or arresting its development; or 2) ameliorating or alleviating the cause of the regression of the disease or disorder.

"Preventing" as used herein refers to the probability of developing a disease, disorder or condition in a subject, who does not have, but is at risk of or susceptible to developing a disease, disorder, or condition.

Preferably, crystalline bempedoic acid or the pharmaceutical composition comprising crystalline bempedoic acid can be used for treating cardiovascular conditions or reducing the risk of cardiovascular conditions. The term "cardiovascular conditions" as used herein refers to diseases of the heart and circulatory system. These diseases are often associated with dyslipoproteinemias and/or dyslipidemias. Cardiovascular diseases which crystalline bempedoic acid and the compositions of the present invention are useful for preventing or treating include but are not limited to arteriosclerosis; atherosclerosis; atherosclerotic cardiovascular disease (ASCVD); stroke; ischemia; endothelium dysfunctions, in particular those dysfunctions affecting blood vessel elasticity; peripheral vascular disease; coronary heart disease; myocardial infarction; cerebral infarction and restenosis.

In a further embodiment, crystalline bempedoic acid or the pharmaceutical composition comprising crystalline bempedoic acid are used for treating a dyslipidemia. The term "dyslipidemia" refers to a disorder that leads to or is manifested by aberrant levels of circulating lipids. To the extent that levels of lipids in the blood are too high, the compositions of the invention are administered to a patient to restore normal levels. Normal levels of lipids are reported in medical treatises known to those of skill in the art. For example, recommended blood levels of LDL, HDL, free triglycerides and others parameters relating to lipid metabolism can be found at the web site of the National Cholesterol Education Program of the National Heart, Lung and Blood Institute (https://www.nhlbi.nih.gov/health-topics/all-publications-and-resources/high-blood-cholesterol-what-you-need-know). At the present time, the recommended level of HDL cholesterol in the blood is above 40 mg/dL; the recommended level of LDL cholesterol in the blood is below 130 mg/dL; and the recommended level of free triglycerides in the blood is less than 150 mg/dL.

Dyslipidemias which crystalline bempedoic acid or the compositions of the present invention are useful for preventing or treating include but are not limited to hyperlipidemia and low blood levels of high density lipoprotein (HDL) cholesterol. In certain embodiments, the hyperlipidemia for prevention or treatment by the bempedoic acid and the pharmaceutical compositions of the present invention is (heterozygous) familial hypercholesterolemia (HeFH); familial combined hyperlipidemia; reduced or deficient lipoprotein lipase levels or activity, including reductions or deficiencies resulting from lipoprotein lipase mutations; hypertriglyceridemia; hypercholesterolemia; high blood levels of urea bodies (e.g. β-OH butyric acid); high blood levels of Lp(a) cholesterol; high blood levels of low density lipoprotein (LDL) cholesterol; high blood levels of very low density lipoprotein (VLDL) cholesterol and high blood levels of non-esterified fatty acids.

In yet a further embodiment, crystalline bempedoic acid or the pharmaceutical composition comprising crystalline bempedoic acid are used for treating patients with hyperlipidemia who are unable to tolerate optimal statin therapy and thus generally have high concentrations of LDL cholesterol. "Statin intolerance" as used herein is defined as the inability to tolerate statins because of muscle pain, weakness, or cramping that began or increased during statin therapy and resolved with statin discontinuation. Preferably, bempedoic acid is used for the treatment in combination with ezetimibe.

In a further embodiment, crystalline bempedoic acid or the pharmaceutical composition comprising crystalline bempedoic acid are used for altering lipid metabolism in a patient, e.g., reducing LDL in the blood of a patient, increasing the ratio of HDL to LDL in the blood of a patient, and inhibiting saponified and/or non-saponified fatty acid synthesis.

Preferably, for the above described treatments, crystalline bempedoic acid or the pharmaceutical compositions of the present invention are administered once or twice daily, preferably once daily.

A further aspect of the present invention is a use of crystalline bempedoic acid for the preparation of amorphous bempedoic acid. In particular, it has been surprisingly found in the present invention that pure amorphous bempedoic acid can be prepared by using crystalline bempedoic acid.

Amorphous bempedoic acid in the context of the present invention means isolated amorphous bempedoic acid which is essentially free from crystalline bempedoic acid, in particular free from crystalline bempedoic acid as described in the present invention. Further, preferably the amorphous bempedoic acid is essentially pure.

"Essentially free" as used herein means that no crystalline bempedoic acid can be detected by X-ray powder diffraction (XRPD) measurement, i.e. no peaks of a crystalline form of bempedoic acid can be observed in an XRPD measurement. Consequently, the amorphous bempedoic acid is preferably phase pure. While a crystalline phase usually produces a distinctive XRPD pattern comprising sharp lines/peaks, amorphous materials produce a broad background signal. Hence, the amorphous bempedoic acid preferably has a phase purity of 90% or more, more preferably of 95% or more, most preferably of 98% or more, as determined via XRPD.

The use of crystalline bempedoic acid for the preparation of amorphous bempedoic acid comprises the steps of:
(I) providing crystalline bempedoic acid as described in the present invention,
(II) dissolving crystalline bempedoic acid of step (i) in a solvent, and
(III) removing the solvent.

Preferably, step (II) of dissolving crystalline bempedoic acid of step (I) in a solvent is carried out at a temperature in the range of 20 to 70°C.

Preferably, the concentration of crystalline bempedoic acid in the solution obtained in step (II) is in the range of 15 to 1000 mg/mL, more preferably in the range of 50 to 750 mg/mL. Most preferably, the solution of the crystalline bempedoic acid obtained in step (II) is saturated.

Further, removing the solvent in step (III) is typically performed by one or more of precipitation and filtration, thermal desolvation, hot melt-extrusion, spray drying, or lyophilization. Preferably, removing the solvent in step (III) is performed by one or more of precipitation and filtration, spray drying, or lyophilization.

In one embodiment of the invention, removing the solvent in step (III) is carried out by adding the solution of step (II) into water and filtrating the precipitate. Preferably, the solvent in step (II) is selected from ethanol, methanol, dioxane, diisopropyl ether, tetrahydrofuran and N,N-dimethylformamide, as well as mixtures thereof.

The filtration of the amorphous bempedoic acid is not limited to specific methods known in the art. The filtration of the amorphous bempedoic acid may therefore be carried out for example by centrifuge filtration.

Moreover, the product obtained in step (III) may further optionally be dried, for example under reduced pressure, typically at room temperature, or heated up to a temperature between 25°C and 70°C.

In a further embodiment, removing the solvent in step (III) is carried out by spray or freeze drying the solution of step (II). Solvents in pure form or mixtures used for spray drying are preferably selected from Biopharmaceutics Classification System (BCS) Class 3 solvent(s), further preferably with a low boiling point, most preferably acetone and dichloromethane. Solvents used for lyophilization are typically selected from dioxane, dimethylsulfoxide (DMSO), tetrahydrofuran, acetic acid, acetone, ethanol, methanol and tert-butanol. Preferably, the solvent in step (II) when step (III) comprises a freeze drying (lyophilization) step is selected from dioxane, tetrahydrofuran, diisopropyl ether, ethanol and methanol, as well as mixtures thereof. More preferably, the solvent in step (II) is selected from dioxane, tetrahydrofuran, diisopropyl ether and mixtures thereof.

The amount of the solvent is not particularly restricted, preferably a 10 to 100-fold amount with respect to the substrate. The time for lyophilization is not particularly restricted, preferably 1 to 240 hours, more preferably 2 to 120 hours.

In a further aspect, the pharmaceutical composition described above comprises amorphous bempedoic acid and is essentially free of bempedoic acid in crystalline form, as determined by XRPD.

The invention shall be illustrated by the following examples.

### EXAMPLES

### XRPD

The XRPD pattern were obtained with a PANalytical X'Pert MPD PRO diffractometer equipped with a θ/θ coupled goniometer in transmission geometry, Cu- Kα1/Kα2 radiation (wavelength 0.15419 nm) with a focusing mirror and a solid state PIXcel detector. The pattern was recorded at a tube voltage of 45 kV and a tube current of 40 mA, applying a step size of 0.013° 2θ with 40s per step (255 channels) in the angular range of 2° to 40° 2θ at ambient conditions (25°C). A typical precision of the 2θ values is in the range of about ± 0.2° 2θ. Thus a diffraction peak that appears at 5.0° 2θ can appear between 4.8 and 5.2° 2θ on most X-ray diffractometers under standard conditions.

### DSC

Differential scanning calorimetry (DSC) was performed on a Mettler Toledo Polymer DSC R instrument. About 3.3 mg of the sample were heated in a 40 µl aluminium pan with a pierced aluminium lid from room temperature (25 °C) to 250°C at a rate of 10°C/min. Nitrogen (purge rate 50 ml/min) was used as purge gas.

### IR

The Fourier transform infrared spectrum (FTIR) was recorded on an MKII Golden Gate™ Single Reflection Diamond ATR (attenuated total reflection) cell with a Bruker Tensor 27 FTIR spectrometer with 4 cm⁻¹ resolution at ambient conditions. To record a spectrum a spatula tip of the sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of about ± 4 cm⁻¹. Thus, an infrared peak that appears at 1716 cm⁻¹ can appear between 1712 and 1720 cm⁻¹ on most infrared spectrometers under standard conditions.

### TGA

Thermogravimetric analysis (TGA) was performed on a Mettler Toledo TGA/DSC 1 instrument. About 11.4 mg of the sample were heated in a 100 µl aluminium pan closed with an aluminium lid. The lid was automatically pierced at the beginning of the measurement. The samples was initially kept at 25°C for 2 ½ minutes and then heated from room temperature to 150°C at a rate of 10°C/min. Nitrogen (purge rate 30 ml/min) was used as purge gas.

### NMR

NMR spectra were recorded with a Bruker Avance 500 MHz, equipped with a BBO probe head, in d6-DMSO.

### Example 1

### 2,2,14,14-Tetramethyl-8-oxopentadecanedioic acid diethyl ester

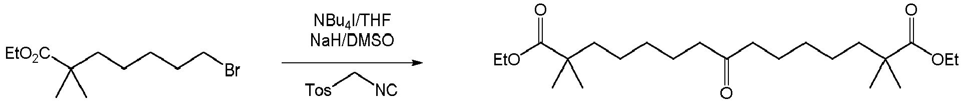

7-Bromo-2,2-dimethylheptanoic acid ethylester (136 g, 512 mmol), Tosylmethylisocyanide (50.0 g, 256 mmol) and tetrabutylammonium iodide (18.8 g, 51 mmol) were dissolved in DMSO (1500 ml). The resulting solution was cooled to 0°C and then NaH, 60% as mineral oil (24.6 g, 370 mmol) was added portion wise keeping the inner temperature below 20°C. After completed addition the mixture was stirred for 16 h at room temperature and then quenched with ice-water (1500 ml). The solution was then extracted with dichloromethane four times (500 ml each) and the combined organic phases were washed with water, brine and then dried over magnesium sulfate. After filtration the organic phase wa acidified with conc. HCl (550 ml) and stirred for 1 h at room temperature. Then the Mixture was diluted with water, the phases were separated, the aqueous phase extracted with dichloromethane and the combined organic phases were washed with sat. NaHCO3-solution and dried over sodium sulfate. After removal of the solvent the crude product was purified via column chromatography (eluent toluene -> dichloromethane) to give 2,2,14,14-tetramethyl-8-oxopentadecanedioic acid diethyl ester in 54% yield (55.5 g) as yellow oil.

### Example 2

### 8-Hydroxy-2,2,14,14-Tetramethylpentadecanedioic acid diethyl ester

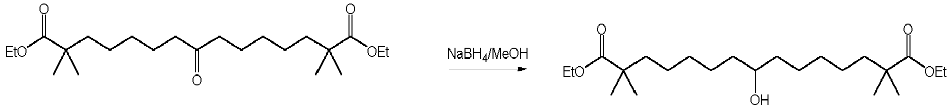

2,2,14,14-tetramethyl-8-oxopentadecanedioic acid diethyl ester (43.5g, 109 mmol) was dissolved in MeOH (400 ml) and cooled to 0°C. To the resulting solution NaBH4 (4.13 g, 109 mmol) was added portion wise keeping the inner temperature below 0°C. The resulting mixture was stirred for 4 h at 0°C and then quenched by the addition of water (400 ml). The solution was then extracted with toluene several times and the combined organic phases were dried over magnesium sulfate. After removal of the solvent the crude product (43.7 g, 100%) was used in the next step without any purification.

### Example 3

### 8-Hydroxy-2,2,14,14-tetramethyl-pentadecanedioic acid

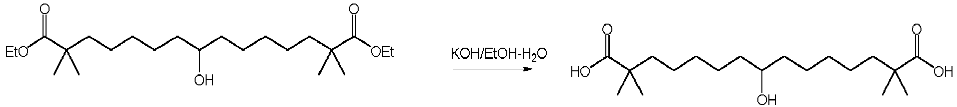

A mixture 8-Hydroxy-2,2,14,14-tetramethyl-pentadecanedioic acid diethyl ester (56.0g, 139mmol) and KOH (29.4g, 524 mmol) in EtOH-water (4:1, 630 ml) was heated to reflux for 4 h. The solvents were removed in vacuo, the residue was acidified with 2N HCl to pH 1. After saturation with brine the product was extracted with H₂CCl₂. After removal of the solvent the crude product was purified via column chromatography (eluent iPr₂O - > Et₂O) and recrystallized from iPr₂O to give bempedoic acid in 58% yield (28.0g) as crystalline material.

## Claims

1. Crystalline bempedoic acid of Formula I 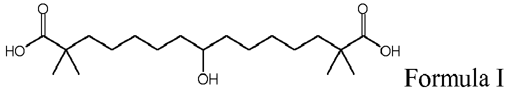 **characterized by** a X-ray powder diffractogram comprising peaks at 10.3, 18.0, 20.3 and 21.8 °2θ as measured according to the description.

2. Crystalline bempedoic acid according to claim 1, **characterized by** a X-ray powder diffractogram further comprising peaks at 15.6, 17.3 and 17.5 °2θ.

3. Crystalline bempedoic acid according to claim 1 or 2, further **characterized by** a melting point of about 85°C, as determined by the onset of the melting endotherm using DSC with a heating rate of 10°C/min.

4. Process of forming bempedoic acid of Formula I 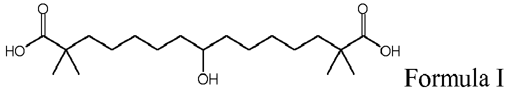 comprising the step of transforming the compound of formula II 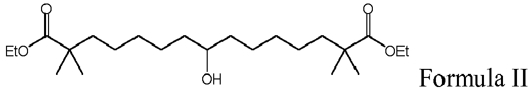 into bempedoic acid of Formula I.

5. Process according to claim 4, wherein the process further comprises the step of obtaining the compound of Formula II from the compound of Formula III

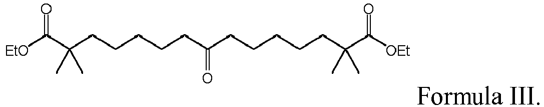

6. Process according to claim 5, wherein the process further comprises the step of obtaining the compound of Formula III from the compound of Formula IV

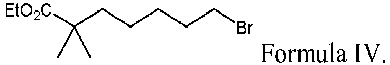

7. Process according to anyone of claims 4 to 6, wherein the step of transforming the compound of formula II into bempedoic acid of Formula I is carried out in the presence of a base or an acid.

8. Process according to claim 7, wherein the step of transforming the compound of formula II into bempedoic acid of Formula I is carried out in the presence of a base.

9. Process according to claim 8, wherein the base is selected from alkali or earth alkali metal hydroxides.

10. Process according to any one of claims 5 to 9, wherein the step of obtaining the compound of Formula II from the compound of Formula III is carried out in the presence of a reducing agent and a solvent.

11. Process according to claim 10, wherein reducing agent is NaBH₄.

12. Process according to claim 10 or 11, wherein the solvent is selected from the group consisting of MeOH, EtOH, THF and mixtures thereof.

13. A pharmaceutical composition comprising crystalline bempedoic acid according to any one of claims 1 to 3.

14. Use of crystalline bempedoic acid according to claims 1 to 3 for the preparation of a pharmaceutical composition.

15. Use of crystalline bempedoic acid according to claims 1 to 3 for the preparation of amorphous bempedoic acid.
